(19) Europäisches Patentamt
European Patent Office
Office européen des brevets

(11) EP 1 877 029 B1

# (12) EUROPEAN PATENT SPECIFICATION

(45) Date of publication and mention of the grant of the patent:
**18.02.2009 Bulletin 2009/08**

(21) Application number: **06742843.3**

(22) Date of filing: **28.04.2006**

(51) Int Cl.:
***A61K 8/11*** *(2006.01)* ***A61K 8/81*** *(2006.01)*
***A61Q 5/00*** *(2006.01)*

(86) International application number:
**PCT/EP2006/004321**

(87) International publication number:
**WO 2006/119960 (16.11.2006 Gazette 2006/46)**

## (54) METHOD OF TREATING HAIR COMPRISING A MICELLAR, SHELL CROSS-LINKED COPOLYMER

VERFAHREN ZUR BEHANDLUNG VON HAAR MIT EINEM MIZELLAREN VERNETZTEN COPOLYMER

PROCEDE DE TRAITEMENT DES CHEVEUX COMPRENANT UN COPOLYMERE MICELLAIRE A ENVELOPPE RETICULEE

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.05.2005 EP 05076062**

(43) Date of publication of application:
**16.01.2008 Bulletin 2008/03**

(73) Proprietors:
- **Unilever PLC**
  **London**
  **EC4Y 0DY (GB)**
  Designated Contracting States:
  **CY GB IE**
- **Unilever N.V.**
  **3013 AL Rotterdam (NL)**
  Designated Contracting States:
  **AT BE BG CH CZ DE DK EE ES FI FR GR HU IS IT LI LT LU LV MC NL PL PT RO SE SI SK TR**

(72) Inventors:
- **KHOSHDEL, Ezat**
  **Bebington, Wirral Merseyside CH63 3JW (GB)**
- **WHOOLEY, Karen, Lynn**
  **Saint Louis, Missouri 63130 (US)**

(74) Representative: **Tansley, Sally Elizabeth**
**Unilever Patent Group**
**Colworth House**
**Sharnbrook**
**Bedford**
**MK44 1LQ (GB)**

(56) References cited:
**WO-A-97/49387** **US-B1- 6 491 903**

- **KOJI ISHIZU,MICHIO YASUDA,TORU TAMURA: "Synthesis of cross-linked core-shell polymer particles by free-radical dispersion copolymerization of 4-vinylpyridine with polystyrene macromonomers in nonaqueous media" JOURNAL OF COLLOID AND INTERFACE SCIENCE, vol. 267, no. 2, 2 July 2003 (2003-07-02), pages 320-325, XP002372181 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 877 029 B1

## Description

### Field of the Invention

The present invention relates to hair care compositions comprising polymers, and to their use in the treatment of hair.

### Background and Prior Art

Hair styling sprays, mousses, gels, shampoos and conditioners frequently contain resins, gums and adhesive polymers to provide a variety of benefits, for example, film-forming ability, thickening, sensory properties and hair shaping and setting.

One of the most common methods for imparting these styling benefits to the hair has been the use of hair fixative agents, such as high molecular weight polymers. The problem with using such agents is that they have a tendency to negatively impact on conditioning attributes such as wet and dry stage clean feel and smoothness. They can make the hair feel stiff and they can leave deposits and result in a sticky feel to the hair.

One way in which this problem has been addressed in the past has been to include conditioning agents, for example silicones and cationic surfactants, in the compositions, to counter the negative effects of the styling agents. Although such conditioning agents do provide substantial improvements in for example the wet and dry combing properties of the hair and in the smoothness of the hair, they tend to have a negative effect on many of the attributes associated with hair body.

WO 02/087522 discloses the incorporation of the small particles covalently grafted with silicone polymer for incorporation into conventional shampoo. The application claims these compositions leads to substantive improvements in the body of the washed and optionally conditioned hair, especially if a subsequent styling regime is followed.

However, there is still a need for a composition that when used to treat hair leaves it feeling well conditioned yet easy to style.

### DEFINITION OF THE INVENTION

The present invention provides a cosmetic method of treating hair which comprises applying to the hair a composition according to the invention.

A further aspect of the invention is the use of a composition of the invention for the cosmetic treatment of hair.

### DETAILED DESCRIPTION OF THE INVENTION

The compositions of the invention comprise a shell cross-linked copolymer, wherein the polymer has a micellar structure. Such shell cross-linked polymers are usually particulate in nature and are known as cross-linked core-shell polymer particulates. These cross-linked core shell particulates preferably have a hydrodynamic diameter, $D_h$, from 1 to 100 nm, more preferably from 5 to 50nm, most preferably from 10 to 30 nm. It is also preferable if the have a dried-state average diameter, $D_{av}$, from 1 to 100 nm, more preferably from 5 to 50 nm, and most preferably from 10 to 30 nm.

It is preferable if the polymeric units comprise a block copolymer, this is especially advantageous for the shell of the polymer, AB block copolymers are particularly preferred.

The Tg of the homo polymeric units (polymeric units before cross-linking occurs) on the exterior shell of the micellar structure is greater than 90°C , preferably greater than 100°C, more preferably greater than 110°C, and most preferably greater than 120°C. The Tg on the interior core of the micellar structure is 110°C or less, preferably 100°C or less , and more preferably less than 50°C and most preferably less than 25 °C.

It is highly preferable if the outer shell of the micellar structure is cross-linked and the core of the micellar structure is not.

The polymer units of the copolymer comprise poly(acrylic acid-co-acrylamide) on the exterior shell of the micellar structure and poly(methyl acrylate) on the internal core of the micellar structure or poly(acrylic acid-co-acrylamide) on the exterior shell of the micellar structure and a polystyrene internal core of the micellar structure.

The cross-linked shell polymers for use with this invention preferably have a molecular weight from 1,000 to 500,000 Da, more preferably from 5,000 to 100,000 Da, and most preferably from 10,000 to 50,000 Da.

Preferably the polymer micelles have structure as shown in figure 1:

The method of manufacture of the polymer micelles used in the present invention are described in J. Mater Chem., 13 2885-2795. Alternative methods of manufacture are described in J. Colloid Interface Sci. 2003 nov15 267(2): 320-5 and WO97/49387

## Compositions of the invention

Cosmetic or personal care compositions of the present invention are formulated into hair care compositions.

Preferably, the compositions are for use in styling human hair and, more preferably, they are packaged and labelled as such.

Compositions of the invention preferably contain the polymer in an amount of from 0.01% to 30% (more preferably from 0.1 to 10%) by weight. Compositions of the invention may, optionally, comprise a fragrance or perfume and/or one or more of the optional additional components described hereinafter.

The carriers and additional components required to formulate such products vary with product type and can be routinely chosen by one skilled in the art. The following is a description of some of these carriers and additional components.

## Hair Treatment Composition Base Formulation

Shampoo compositions preferably comprise one or more cleansing surfactants, which are cosmetically acceptable and suitable for topical application to the hair. Further surfactants may be present as emulsifiers.

Suitable cleansing surfactants, are selected from anionic, amphoteric and zwitterionic surfactants, and mixtures thereof. The cleansing surfactant may be the same surfactant as the emulsifier, or may be different.

## Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate(n)EO, (where n ranges from 1 to 3).

The total amount of anionic cleansing surfactant in shampoo compositions of the invention is generally from 5 to 30, preferably from 6 to 20, more preferably from 8 to 16 wt% of the total composition.

## Co-surfactant

The shampoo composition can optionally include co-surfactants, preferably an amphoteric or zwitterionic surfactant, which can be included in an amount ranging from 0 to about 8, preferably from 1 to 4 wt%.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred co-surfactant is a nonionic surfactant, which can be included in an amount ranging from 0 to 8 wt%, preferably from 2 to 5 wt% of the total composition.

For example, representative nonionic surfactants that can be included in shampoo compositions of the invention include condensation products of aliphatic ($C_8$ - $C_{18}$) primary or secondary linear or branched chain alcohols or phenols with alkylene oxides, usually ethylene oxide and generally having from 6 to 30 ethylene oxide groups.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

$$RO - (G)_n$$

wherein R is a branched or straight chain $C_5$ to $C_{20}$alkyl or alkenyl group, G is a saccharide group and n is from 1 to 10.

Other sugar-derived nonionic surfactants which can be included in shampoo compositions of the invention include the $C_{10}$-$C_{18}$ N-alkyl ($C_1$-$C_6$) polyhydroxy fatty acid amides, such as the $C_{12}$-$C_{18}$ N-methyl glucamides, as described for example in WO 92 06154 and US 5 194 639, and the N-alkoxy polyhydroxy fatty acid amides, such as $C_{10}$-$C_{18}$ N-(3-methoxypropyl) glucamide.

The shampoo composition can also optionally include one or more cationic co-surfactants included in an amount ranging from 0.01 to 10, more preferably from 0.05 to 5, most preferably from 0.05 to 2 wt% of the total composition. Useful cationic surfactants are described herein in relation to conditioner compositions.

The total amount of surfactant (including any co-surfactant, and/or any emulsifier) in shampoo compositions of the invention is generally from 5 to 50, preferably from 5 to 30, more preferably from 10 to 25 wt%.

**Cationic Deposition Polymer**

A cationic polymer is a preferred ingredient, especially in shampoo compositions of the invention.

The cationic polymer may be a homopolymer or be formed from two or more types of monomers. The molecular weight of the polymer will generally be between 5 000 and 10 000 000 Dalton, typically at least 10 000 and preferably from 100 000 to 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic deposition polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic deposition polymers can comprise mixtures of monomer units derived from amine- and/or quaternary ammonium-substituted monomer and/or compatible spacer monomers.

Suitable cationic deposition polymers include, for example:

- copolymers of 1-vinyl-2-pyrrolidine and 1-vinyl-3-methylimidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);

- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);

- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;

- mineral acid salts of amino-alkyl esters of homo-and copolymers of unsaturated carboxylic acids having from 3 to 5 carbon atoms, (as described in U.S. Patent 4,009,256);

- cationic polyacrylamides(as described in WO95/22311).

Other cationic deposition polymers that can be used include cationic polysaccharide polymers, such as cationic cellulose derivatives, cationic starch derivatives, and cationic guar gum derivatives. Suitably, such cationic polysaccharide polymers have a charge density from 0.1 to 4 meq/g.

Cationic polysaccharide polymers suitable for use in compositions of the invention include those of the formula:

$$A\text{-}O\text{-} [R\text{-}N^{+} (R^{1}) (R^{2}) (R^{3}) X^{-}],$$

wherein: A is an anhydroglucose residual group, such as a starch or cellulose anhydroglucose residual. R is an alkylene, oxyalkylene, polyoxyalkylene, or hydroxyalkylene group, or combination thereof. $R^1$, $R^2$ and $R^3$ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in $R^1$, $R^2$ and $R^3$) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic deposition polymer is selected from cationic cellulose and cationic guar derivatives. Particularly preferred cationic polymers are JAGUAR C13S, JAGUAR C15, JAGUAR C17 and JAGUAR C16 and JAGUAR C162.

The cationic deposition polymer will generally be present in compositions of the invention at levels of from 0.01 to 5, preferably from 0.02 to 1, more preferably from 0.04 to 0.5 percent by weight of the composition.

## Conditioning Surfactant

Conditioner compositions usually comprise one or more conditioning surfactants which are cosmetically acceptable and suitable for topical application to the hair.

Suitable conditioning surfactants are selected from cationic surfactants, used singly or in a mixture.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

$$[N(R_1) (R_2) (R_3) (R_4)1^{+} (X)$$

in which $R_1$, $R_2$, $R_3$, and $R_4$ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

The most preferred cationic surfactants for conditioner compositions of the present invention are monoalkyl quaternary ammonium compounds in which the alkyl chain length is C16 to C22.

Examples of suitable cationic surfactants include quaternary ammonium compounds, particularly trimethyl quaternary compounds.

Preferred quaternary ammonium compounds include cetyltrimethylammonium chloride, benzyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen (e.g. , bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

Salts of primary, secondary, and tertiary fatty amines are also suitable cationic surfactants. The alkyl groups of such amines preferably have from 12 to 22 carbon atoms, and can be substituted or unsubstituted.

Particularly useful are amido substituted tertiary fatty amines, in particular tertiary amines having one $C_{12}$ to $C_{22}$ alkyl or alkenyl chain. Such amines, useful herein, include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, rachidamidopropyldimethylamine, arachid amidopropyldiethylamine, rachidamidoethyldiethylamine, arachidamidoethyldimethylamine, diethylaminoethylstearamide. Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

These amines are typically used in combination with an acid to provide the cationic species. The preferred acid useful herein includes L- glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal, et al., issued June 23, 1981.

The molar ratio of protonatable amines to $H^+$ from the acid is preferably from about 1:0.3 to 1:1.2, and more preferably from about 1:0.5 to about 1:1.1.

In the conditioners of the invention, the level of cationic surfactant is preferably from 0.01 to 10, more preferably 0.05 to 5of the total composition (is this low - I think our current conditioners are possibly higher than 2wt% than this already. I would like to keep range 0.05 to 5 as tightest spec).

The cationic surfactants detailed in this section are also suitable for use in the aspect of the invention wherein a cationic surfactant is intimately mixed with the thermotropic mesogenic material and with oily conditioning material prior to the incorporation of the conditioning material into the final hair conditioning composition

**Fatty Materials**

Conditioner compositions of the invention preferably additionally comprise fatty materials. The combined use of fatty materials and cationic surfactants in conditioning compositions is believed to be especially advantageous, because this leads to the formation of a structured phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners of the invention is suitably from 0.01 to 15, preferably from 0.1 to 10 wt%, The weight ratio of cationic surfactant to fatty alcohol is suitably from 10:1 to 1:10, preferably from 4:1 to 1:8, optimally from 1:1 to 1:7, for example 1:3.

**Suspending Agents**

In one preferred embodiment, the shampoo compositions of this invention further comprises from 0.1 to 5 wt% of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

## Styling polymers

In some instances a further styling polymer may also be present.

The hair styling polymer if present is preferably present in the compositions of the invention in an amount of from 0.001% to 10% by weight, more preferably from 0.1% to 10% by weight, such as from 1% to 8% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

The amount of the polymer may range from 0.5 to 10%, preferably 0.75 to 6% by weight based on total weight of the composition.

Examples of anionic hair styling polymers are:

copolymers of vinyl acetate and crotonic acid;

terpolymers of vinyl acetate, crotonic acid and a vinyl ester of an alpha-branched saturated aliphatic monocarboxylic acid such as vinyl neodecanoate;

copolymers of methyl vinyl ether and maleic anhydride (molar ratio about 1:1) wherein such copolymers are 50% esterified with a saturated alcohol containing from 1 to 4 carbon atoms such as ethanol or butanol;

acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.

The polymer may also contain grafted silicone, such as polydimethylsiloxane.

Specific examples of suitable anionic hair styling polymers are:

RESYN® 28-2930 available from National Starch (vinyl acetate/crotonic acid/vinyl neodecanoate copolymer);

ULTRAHOLD® 8 available from BASF (CTFA designation Acrylates/acrylamide copolymer);

the GANTREZ®ES series available from ISP corporation esterified copolymers of methyl vinyl ether and maleic anhydride).

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N-vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:

copolymers of N-vinylpyrrolidone and dimethylaminoethyl methacrylate, available from ISP Corporation as Copolymer 845, Copolymer 937 and Copolymer 958;

copolymers of N-vinylpyrrolidone and dimethylaminopropylacrylamide or methacrylamide, available from ISP Corporation as Styleze® CC10;

copolymers of N-vinylpyrrolidine and dimethylaminoethyl methacrylate;

copolymers of vinylcaprolactam, N-vinylpyrrolidone and dimethylaminoethylmethacrylate;

Polyquaternium-4 (a copolymer of diallyldimonium chloride and hydroxyethylcellulose);

Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;

Polyquaternium-16 (formed from methylvinylimidazolium chloride and vinylpyrrolidone), available from BASF as Luviquat® FC 370, FC 550, FC 905 and HM-552;

Polyquaternium-46 (prepared by the reaction of vinylcaprolactam and vinylpyrrolidone with methylvinylimidazolium methosulphate), available from BASF as Luviquat®Hold.

Examples of suitable naturally-derived polymers include shellac, alginates, gelatins, pectins, cellulose derivatives and chitosan or salts and derivatives thereof. Commercially available examples include Kytamer® (ex Amerchol) and Amaze® (ex National Starch).

**Adjuvants**

The compositions of the present invention may also contain adjuvants suitable for hair care. Generally such ingredients are included individually at a level of up to 2, preferably up to 1 wt% of the total composition.

Suitable hair care adjuvants, include amino acids and ceramides.

The invention also involves a method of styling hair by applying thereto a styling composition as is hereinabove described.

The following non-limiting Examples further illustrate the preferred embodiments of the invention. All percentages referred to are by weight based on total weight unless otherwise indicated.

**EXAMPLES**

The following Examples were prepared according to J. Mater Chem., 13 2885-2795**.**

**Example 1**

0.02 wt % - 50% cross-linked poly(acrylic acid-co-acrylamide) shell, poly(methyl acrylate) core, n is 164, m is 93 (n and m are as defined in figure 1).

**Example 2**

0.02 wt%- 50% cross-linked poly(acrylic acid-co-acrylamide) shell, polystyrene core. n is 650, m is 93 (N and m are as defined in figure 1).

**Example A**

0.02 wt% Ludox W50 in an aqueous solution. (Ludox is colloidal $Al_2O_3$ ex Grace )

**Example B**

0.02 wt% 1785 Silicone

| Polymer | Glass transition temperature (oC) |
|---|---|
| Polyacrylic acid | 105 |
| Polyacrylamide | 165 |
| Polymethylacrylate | 10 |
| Polystyrene | 100 |

**Evaluation**

The above Examples were tested using the Protocol for Instron Pullthrough Method

The Instron pullthrough technique was developed as a method of measuring tactile attributes relating to "bods' .

2g/10" hair switches were used. Five are used per product and five pull-throughs are recorded per switch. Each set of five switches is treated with test product together, rinsed and dried.

A single switch was hung from the clamp so that the aperture was in line with the first half an inch. The aperture was reduced to 7.5mm and the position adjusted so that the switch hung in the centre. The force cell is zeroed and the test started. The switch was pulled upwards and the forces recorded by the Instron force cell. The peak value was noted. The aperture was opened, the switch returned to its starting position and the test repeated 4 times on that switch. The test was repeated for all five switches per product tested and the results recorded.

| Material | Peak Force |
|---|---|
| Example 1 | 109.28 |
| Example 2 | 161.91 |
| Example A | 140.96 |
| Example B | 95.64 |

Using the same treatment method described above for the hair switches a ranking panel test was performed to observe the feel properties of the different materials using the scale 0-100. Attributes asked included, smoothness, ease of combing and coated feel.

| Material | Smooth | Coated | Ease of combing |
|---|---|---|---|
| Example 1 | 650 | 590 | 490 |
| Example 2 | 450 | 500 | 320 |
| Example A | 400 | 650 | 350 |

(continued)

| Material | Smooth | Coated | Ease of combing |
|---|---|---|---|
| Example B | 650 | 640 | 700 |
| Smooth - a higher score means the hair feels smoother. Combing - a higher score means the hair is easier to comb. Coated - a higher score means the hair feels more coated - a negative attribute. | | | |

It can thus be seen that compositions according to the invention gave an overall affect of acceptable volume scores without detrimental effects on the overall sensory performance

The following formulations were prepared:

### Example 3

A styling gel is formulated as follows:

| Material | wt % |
|---|---|
| Polymer of any of Examples 1 to 2 | 0.8 |
| Carbopol 980 | 0.4 |
| Water | to 100% |
| Sepicide LD | 0.4 |
| Sodium hydroxide (8% 2M) | 0.1 |
| Ethanol | 10.0 |
| Cremaphor RH410 | 0.4 |
| Jaguar HP-105 | 0.2 |
| Perfume | 0.15 |

### Example 4 - Shampoo Formulation

| | | |
|---|---|---|
| **Trade Name** | **Chemical Name** | wt % |
| Texapon N701 | SodiumLaurylEth erSulfate 1-EO | 12.00 |
| Tegobetain CK | Cocoamidopropyl betaine | 1.60 |
| Carbopol 980 | Carboxymethylce llulose | 0.40 |
| Jaguar C13S | Cationic guar polymer | 0.10 |
| Sodium Chloride | Sodium Chloride | 1.00 |
| Polymer of Examples 2, 3 | | 0.5 |
| Water and minors | Water | To 100% |

### Example 5 Conditioner Formulation

| | wt% |
|---|---|
| PEG-2 oleamonium chloride & propylene glycol | 2.0 |
| Cetyl/stearyl alcohol | 8.0 |
| Disodium EDTA | 0.1 |
| DMDM hydantoin 55%active | 0.1 |
| Silicone DC245 | 1.8 |
| Polymer micelle of Example 1 | 0.2 |
| Water and minors | to 100 |

**Claims**

**1.** A method of treating hair which comprises applying to the hair a hair care composition comprising :

i) a micellar, shell cross-linked copolymer comprising polymeric units in its centre and cross-linked polymeric units on its exterior; the polymer units in its centre having a Tg of less than 110°C and its homopolymeric units on its exterior shell having a Tg of greater than 90°C; wherein the polymer units of the copolymer comprise poly (acrylic acid-co-acrylamide) on the exterior shell of the micellar structure and poly(methyl acrylate) on the internal core of the micellar structure or poly(acrylic acid-co-acrylamide) on the exterior shell of the micellar structure and a polystyrene internal core of the micellar structure and
ii) a cosmetically acceptable diluent or carrier.

**2.** A hair method according to claim 1 in which the exterior shell comprises a cross-linked copolymers and the interior core is not cross linked.

**3.** A hair method according to claims 1 or 2 in which the polymeric units comprise a block copolymer.

**4.** A hair method according to claim 3 in which the block copolymer comprises a AB block copolymer.

**5.** A hair method according to any preceding claim in which the Tg of the polymer units on the exterior shell of the micellar structure is greater than 110°C and the Tg on the interior core of the micellar structure is 50°C.

**6.** A hair method according to any preceding claim in which the shell cross-linked copolymer is a nanoparticulate.

**7.** A hair method according to any preceding claim in which the molecular weight of the shell cross- linked copolymer is from 1,000 to 500,000.

**8.** A hair method according to any preceding claim wherein the cosmetically acceptable diluent or carrier of the composition is selected from water, ethanol and mixtures thereof.

**9.** A hair method according to any preceding claim, wherein the composition further comprising a fragrance or perfume.

**10.** A hair method according to any preceding claim wherein the composition is a hair styling composition.

**11.** A hair method according to any preceding claim wherein the composition comprises from 0.001 to 10% by weight of the polymer.

**12.** A hair method according to any preceding claim wherein the composition further comprising an additional hair styling polymer.

**13.** A hair method according to any preceding claim wherein the composition further comprising from 0.01% to 7.5% by weight of a surfactant.

**14.** Use of a composition as described in any one of Claims 1 to 13 for the cosmetic treatment of hair.

**Patentansprüche**

1. Verfahren zur Behandlung von Haar, umfassend Auftragen auf das Haar einer Haarpflegezusammensetzung, umfassend:

   i) ein micellares, hüllenvernetztes Copolymer, das Polymer-Einheiten in seiner Mitte und vernetzte Polymer-Einheiten an seiner Außenseite umfasst; wobei die Polymer-Einheiten in seiner Mitte eine Tg von kleiner als 110 °C haben und seine Homopolymer-Einheiten an seiner äußeren Hülle eine Tg von höher als 90 °C haben; wobei die Polymer-Einheiten des Copolymers Poly(acrylsäure-co-acrylamid) an der äußeren Hülle der micellaren Struktur und Poly(methylacrylat) am inneren Kern der micellaren Struktur oder Poly(acrylsäure-co-acrylamid) an der äußeren Hülle der micellaren Struktur und einen Polystyrol-Innenkern der micellaren Struktur umfassen, und
   ii) ein kosmetisch verträgliches Verdünnungsmittel oder einen kosmetisch verträglichen Träger.

2. Verfahren zur Behandlung von Haar gemäß Anspruch 1, wobei die äußere Hülle ein vernetztes Copolymer umfasst und der innere Kern nicht vernetzt ist.

3. Verfahren zur Behandlung von Haar gemäß Anspruch 1 oder 2, wobei die Polymer-Einheiten ein Block-Copolymer umfassen.

4. Verfahren zur Behandlung von Haar gemäß Anspruch 3, wobei das Blockcopolymer ein AB-Block-Copolymer umfasst.

5. Verfahren zur Behandlung von Haar gemäß einem vorangehenden Anspruch, bei dem die Tg der Polymer-Einheiten an der äußeren Hülle der micellaren Struktur höher als 110 °C ist und die Tg am inneren Kern der micellaren Struktur 50 °C ist.

6. Verfahren zur Behandlung von Haar gemäß einem vorangehenden Anspruch, wobei das hüllenvernetzte Copolymer ein nanopartikuläres Material ist.

7. Verfahren zur Behandlung von Haar gemäß einem vorangehenden Anspruch, wobei das Molekulargewicht des hüllenvernetzten Copolymers 1 000 bis 500 000 ist.

8. Verfahren zur Behandlung von Haar gemäß einem vorangehenden Anspruch, wobei das kosmetisch verträgliche Verdünnungsmittel oder der kosmetisch verträgliche Träger der Zusammensetzung aus Wasser, Ethanol und Gemischen davon ausgewählt ist.

9. Verfahren zur Behandlung von Haar gemäß einem vorangehenden Anspruch, wobei die Zusammensetzung außerdem einen Duftstoff oder ein Parfüm umfasst.

10. Verfahren zur Behandlung von Haar gemäß einem vorangehenden Anspruch, wobei die Zusammensetzung eine Haarstyling-Zusammensetzung ist.

11. Verfahren zur Behandlung von Haar gemäß einem vorangehenden Anspruch, wobei die Zusammensetzung 0,001 bis 10 Gewichts-% des Polymers umfasst.

12. Verfahren zur Behandlung von Haar gemäß einem vorangehenden Anspruch, wobei die Zusammensetzung außerdem ein zusätzliches Haarstyling-Polymer umfasst.

13. Verfahren zur Behandlung von Haar gemäß einem vorangehenden Anspruch, wobei die Zusammensetzung außerdem 0,01 Gewichts-% bis 7,5 Gewichts-% eines oberflächenaktiven Mittels umfasst.

14. Verwendung einer Zusammensetzung, wie sie in einem der Ansprüche 1 bis 13 beschrieben ist, für die kosmetische Behandlung von Haar.

## Revendications

1. Méthode de traitement des cheveux comprenant l'application sur les cheveux d'une composition de soins capillaire comprenant :

   i) un copolymère micellaire réticulé en coquille comprenant des motifs polymères en son centre et des motifs polymères réticulés sur son extérieur ; les motifs polymères en son centre possédant une Tg inférieure à 110°C et ses motifs homopolymères sur sa coquille externe possédant une Tg supérieure à 90°C ; dans lequel les motifs polymères du copolymère comprennent un poly(acide acrylique-co-acrylamide) sur la coquille extérieure de la structure micellaire et un poly(acrylate de méthyle) sur le coeur interne de la structure micellaire ou un poly(acide acrylique-co-acrylamide) sur la coquille extérieure de la structure micellaire et un coeur interne en polystyrène de la structure micellaire, et
   ii) un diluant ou support acceptable sur le plan cosmétique.

2. Méthode de traitement des cheveux selon la revendication 1, dans laquelle la coquille externe comprend des copolymères réticulés et le coeur interne n'est pas réticulé.

3. Méthode de traitement des cheveux selon les revendications 1 ou 2, dans laquelle les motifs polymères comprennent un copolymère séquencé.

4. Méthode de traitement des cheveux selon la revendication 3, dans laquelle le copolymère séquencé comprend un copolymère séquencé AB.

5. Méthode de traitement des cheveux selon l'une quelconque des revendications précédentes, dans laquelle la Tg des motifs polymères sur la coquille externe de la structure micellaire est supérieure à 110°C et la Tg sur le coeur interne de la structure micellaire est de 50°C.

6. Méthode de traitement des cheveux selon l'une quelconque des revendications précédentes, dans laquelle le copolymère réticulé en coquille est une nanoparticule.

7. Méthode de traitement des cheveux selon l'une quelconque des revendications précédentes, dans laquelle le poids moléculaire du copolymère réticulé en coquille est de 1 000 à 500 000.

8. Méthode de traitement des cheveux selon l'une quelconque des revendications précédentes, dans laquelle le diluant ou support acceptable sur le plan cosmétique de la composition est choisi parmi l'eau, l'éthanol et leurs mélanges.

9. Méthode de traitement des cheveux selon l'une quelconque des revendications précédentes, dans laquelle la composition comprend en outre une fragrance ou un parfum.

10. Méthode de traitement des cheveux selon l'une quelconque des revendications précédentes, dans laquelle la composition est une composition coiffante.

11. Méthode de traitement des cheveux selon l'une quelconque des revendications précédentes, dans laquelle la composition comprend 0,001 à 10 % en poids du polymère.

12. Méthode de traitement des cheveux selon l'une quelconque des revendications précédentes, dans laquelle la composition comprend en outre un polymère coiffant supplémentaire.

13. Méthode de traitement des cheveux selon l'une quelconque des revendications précédentes, dans laquelle la composition comprend en outre 0,01 % à 7,5 % en poids d'un tensioactif.

14. Utilisation d'une composition selon l'une quelconque des revendications 1 à 13, pour le traitement cosmétique des cheveux.

# Fig.1.

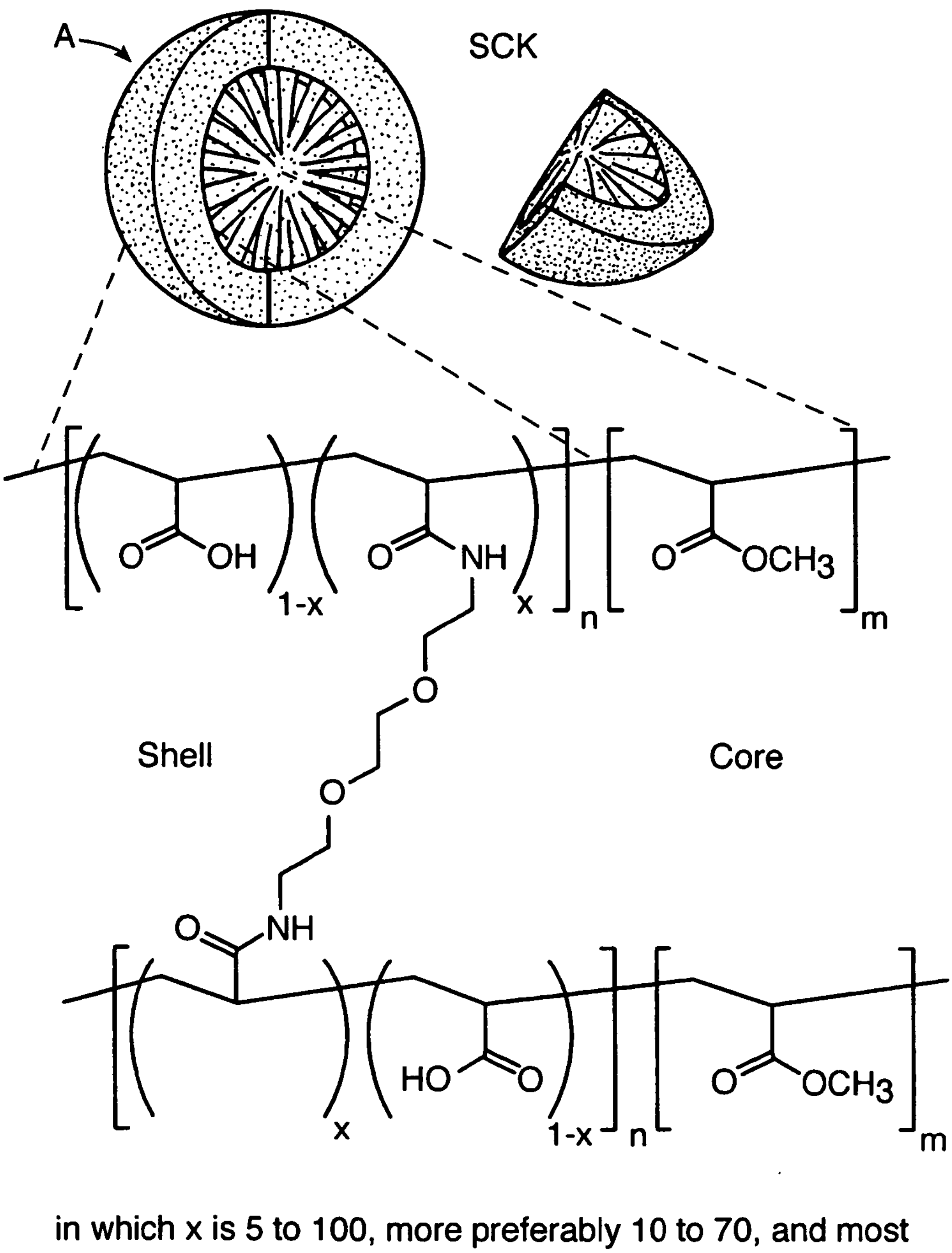

in which x is 5 to 100, more preferably 10 to 70, and most preferably 20 to 50; m is preferably from 10 to 5000, more preferably 50 to 1000, most preferably 100 to 500; and n is preferably from 10 to 5000, more preferably 50 to 1000, most preferably 100 to 500.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02087522 A
- WO 9749387 A
- WO 9206154 A
- US 5194639 A
- US 4009256 A
- WO 9522311 A
- US 3962418 A
- US 3958581 A
- US 4275055 A, Nachtigal
- EP 0619111 A
- US 5000955 A
- US 3822238 A
- US 4156066 A
- US 4156067 A
- US 4255550 A
- US 4743673 A
- EP 0240350 A
- WO 9631188 A


### Non-patent literature cited in the description


- *J. Mater Chem.,* vol. 13, 2885-2795
- *J. Colloid Interface Sci.,* 15 November 2003, vol. 267 (2), 320-5